# EUROPÄISCHE PATENTANMELDUNG

(11) **EP 1 800 672 A1**
(43) Veröffentlichungstag der Anmeldung: **27.06.2007**
(21) Anmeldenummer: 05028387.8
(22) Anmeldetag: 23.12.2005
(51) Int. Cl.: A61K 31/00, A61K 31/198, A61K 45/06, A61P 17/00

(54) **Pharmazeutische Zusammensetzung und deren Verwendung zur Behandlung von Dermatosen**

(71) Anmelder: Dr. August Wolff GmbH & Co., 33611 Bielefeld (DE)
(72) Erfinder: Fischer, Matthias, 06120, Halle (DE)
(74) Vertreter: Grünecker, Kinkeldey, Stockmair & Schwanhäusser Anwaltssozietät

(57) **Zusammenfassung**

Die vorliegende Erfindung betrifft eine pharmazeutische Zusammensetzung und deren Verwendung zur Behandlung von Dermatosen, die durch ein gestörtes Verhornungsmuster charakterisiert sind.

## Beschreibung

Die vorliegende Erfindung betrifft eine pharmazeutische Zusammensetzung und deren Verwendung zur Behandlung von Dermatosen, die durch ein gestörtes Verhornungsmuster charakterisiert sind.

Die Haut stellt die äußere Begrenzung des Individuums zu seiner Umgebung dar und ist eine Barriere gegen eine Vielzahl von schädlichen Umwelteinflüssen. Ihr äußerster Anteil, die Epidermis, ist ein geschichtetes Plattenepithel ektodermaler Herkunft, das zu ca. 90% aus Keratinozyten besteht, die aus dem sich stetig regenerierenden Stratum basale hervorgehen. Ihm folgt das 2-6 Zelllagen dicke Stratum spinosum, in dem eine allmähliche Zunahme des Zellvolumens zu beobachten ist. In dem sich anschließenden Stratum granulosum beschleunigt sich die Differenzierung der Keratinozyten, die mit dem Verschwinden von funktionellen Zellorganellen und dem Beginn der terminalen Verhornung einhergeht. Die Zellen enthalten zunehmend basophile Keratohyalingranula und sind deutlich abgeflacht. Als oberflächlichste Schicht der Epidermis schließt sich dann das Stratum corneum (Homschicht) an, das aus flachen, fest gepackten, kernlosen Homzellen besteht, die dicht mit Tonofilamenten und einer amorphen Matrix gefüllt sind. Die Umwandlung von vitalen Keratinozyten des oberen Stratum granulosum in die Hornschicht ist ein abrupter Vorgang, der sich lediglich über Stunden erstreckt (Jung E: Dermatologie. 4. Aufl. Hippokrates, Stuttgart, 1998, S. 18-22).

Dieser Prozess der Differenzierung der Keratinozyten unterliegt einem stetigen Einfluss von Wachstumsfaktoren, Zytokinen, Fettsäuren und Ceramiden (Schneider IM, Wohlrab W, Neubert R: Hautarzt 1997; 48: 303-310). Die epidermale Differenzierung ist ein Prozess, der mit der Expression spezifischer Strukturproteine verbunden ist. Zu ihnen gehören Involucrin, Loricrin, Filaggrin und diverse andere Proteine, die enzymatisch synthetisiert, verknüpft, abgebaut und prozessiert werden. Einen entscheidenden Einfluss bei diesem als terminale epidermale Differenzierung bezeichneten Vorgang hat die intrazelluläre Calciumkonzentration (Bikle et al. Molecular and Cellular Endocrinology 2001, 177, 161-171; Landsdown ABG: Wound Rep Reg 2002, 10, 271-285). Daher sind Faktoren, die Einfluss auf die intrazelluläre Calciumkonzentration nehmen können, für die Differenzierung, aber auch die Proliferation von Keratinozyten von besonderem Interesse. Hierzu gehören insbesondere Rezeptoren, wie der N-Methyl-D-Aspartat-Rezeptor (NMDA-Rezeptor), die nach ihrer Aktivierung als transmembranöser Calciumkanal fungieren.

Die Existenz von Glutamatrezeptoren vom Typ der N-Methyl-D-Aspartat-Rezeptoren (NMDA-Rezeptoren) in Keratinozyten konnte nachgewiesen werden (Fischer et al. Arch. Dermatol. Res. 2004, 296, 157-162; Fischer et al. Experimental Dermatology 2004, 13, 512-519). Dabei fand sich in der gesunden Epidermis eine Expression von NMDA-Rezeptoren in allen vitalen Schichten der Epidermis mit besonderer Betonung des Stratum granulosum. Dieses Verteilungsmuster korreliert dabei sehr gut mit dem bekannten intraepidermalen Calciumgradienten, der durch einen abrupten Anstieg im Stratum granulosum gekennzeichnet ist (Menon und Elias: Arch. Dermatol. 1991; 127: 57-63). Dieser Calcium-Peak wird als wesentliches Signal für die Differenzierung der Keratinozyten angesehen. Darüber hinaus korreliert das gefundene Expressionsmuster der NMDA-Rezeptoren mit der intraepidermalen Konzentration von L-Glutamat, dem physiologischen Agonisten am NMDA-Rezeptor (Fuziwara et al. J. Invest. Dermatol. 2003, 120, 1023-1029).

In ersten Untersuchungen zur Funktion keratinozytärer NMDA-Rezeptoren fanden sich Hinweise auf eine differenzierungsfördernde Wirkung. So führte in den Untersuchungen die Blockade des NMDA-Rezeptors mit dem selektiven Antagonisten MK-801 bei kultivierten Keratinozyten (HaCaT-Zellen) zur verminderten Expression der Differenzierungsmarker Filaggrin und Cytokeratin 10 (Fischer et al. Arch. Dermatol. Res. 2004, 296, 157-162; Fischer et al. Experimental Dermatology 2004, 13, 512-519). In einer weiteren Studie wurde bei Nacktmäusen eine Verminderung der Barrierefunktion der Epidermis nach Applikation des selektiven Rezeptoragonisten N-Methyl-D-Aspartat (NMDA) beschrieben und daraus ein möglicher Einfluss auf die Proliferation von Keratinozyten abgeleitet (Fuziwara et al. J. Invest. Dermatol. 2003, 120, 1023-1029). Bei dieser Untersuchung bestehen jedoch methodische Mängel durch die Verwendung ungepufferter Lösungen, so dass der beobachtete Effekt allein durch den sauren pH von NMDA bedingt sein kann.

Aufgabe der vorliegenden Erfindung ist es daher, eine pharmazeutische Zusammensetzung zur Verfügung zu stellen, die zur Behandlung von Dermatosen mit gestörtem Verhomungsmuster eingesetzt werden kann. Es soll die gestörte Proliferation und insbesondere die veränderte Differenzierung der Keratinozyten, welche diesen Krankheitsbildem zugrunde liegt, reguliert werden. Weiterhin sollten möglichst wenig Nebenwirkungen durch das Medikament bzw. durch die Behandlung verursacht werden.

Diese Aufgabe wird durch die Verwendung mindestens eines N-Methyl-D-Aspartat-Rezeptor-Agonisten zur Herstellung eines Medikaments zur Behandlung von Dermatosen, die durch ein gestörtes Verhornungsmuster charakterisiert sind, gelöst. In einer bevorzugten Ausführungsform enthält die Zusammensetzung zusätzlich mindestens einen N-Methyl-D-Aspartat-Rezeptor-Co-Agonisten. Ebenso bevorzugt sind Zusammensetzungen welche mindestens ein Zn²⁺-chelatisierendes Agens enthalten. Besonders bevorzugt sind Zusammensetungen welche sowohl mindestens einen N-Methyl-D-Aspartat-Rezeptor-Co-Agonisten als auch mindestens ein Zn²⁺-chelatisierendes Agens enthalten.

Von den oben bevorzugten und besonders bevorzugten Zusammensetzungen sind diejenigen ganz besonders bevorzugt, welche weiterhin mindestens ein Ca²⁺-lonen spendendes Agens enthalten. Ebenfalls ganz besonders bevorzugt werden Zusammensetzungen, welche zusätzlich mindestens eine differenzierungsfördernde Substanz enthalten. In den ganz besonders bevorzugten Zusammensetzungen können auch sowohl mindestens ein Ca²⁺-Ionen spendendes Agens als auch mindestens eine differenzierungsfördernde Substanz enthalten sein.

Die Zusammensetzung wird entsprechend der Indikation und der Behandlungsart appliziert, wobei die topischen und subkutanen Applikationsformen bevorzugt werden.

Erfindungsgemäß erfolgt die Wiederherstellung der Homöostase in der erkrankten Haut durch die Steuerung der intrazellulären Calcium-Konzentration in Hautzellen. Dieser Effekt wird durch die entsprechende Applikation des beanspruchten Mittels erzielt. Hierbei werden transmembranöse Calciumkanäle (NMDA-Rezeptoren) der Hautzellen durch einen NMDA-Rezeptor-Agonisten aktiviert, wodurch die intrazelluläre Calcium-Konzentration gezielt erhöht wird. Dabei werden Zusammensetzungen des beanspruchten Mittels bevorzugt, welche ein physiologisch wirksames Ca²⁺-spendendes Agens enthalten. Dieses dient der Sicherung einer ausreichend hohen extrazellulären Calcium-Konzentration in der Epidermis, so dass ausreichend Calcium durch den aktivierten NMDA-Rezeptor nach intrazellulär strömen kann.

Ionenkanäle, wie im Falle des NMDA-Rezeptors, sind Transmembranproteine mit Poren, die es Ionen ermöglichen, die Zellmembran zu durchqueren. Im Unterschied zu dem aktiven Transport in Ionen-Transportern erfolgt der Transport von Ionen in lonenkanälen passiv, dem elektrochemischen Gradienten entsprechend. Ionenkanäle sind gesteuert, d. h. ihre Leitfähigkeit für Ionen hängt von bestimmten Signalen ab. Eine große Klasse von Ionenkanälen wird durch Liganden aktiviert, d.h. durch Moleküle, die als Botenstoffe fungieren. Zu diesen Ionenkanälen zählen die NMDA-Rezeptoren, die nach ihrem Agonisten N-Methyl-D-Aspartat benannt sind, welche im wissenschaftlichen Experiment zur Öffnung des Kanals geführt haben. Mit dem Terminus "NMDA-Rezeptor" ist ein lonenkanal in der Zellmembran gemeint, der nach Bindung eines Liganden selektiv leitfähig für bestimmte Ionen wird. In den Keratinozyten ist der NMDA-Rezeptor bei seiner Aktivierung für Ca²⁺-lonen durchlässig. Wichtig sind dabei sowohl die Rezeptor- wie auch die lonenkanal-Untereinheiten. Die NMDA-Rezeptoren gehören zur Familie der ionotropen Glutamatrezeptoren und enthalten sieben Untereinheiten: NR1, NR2A bis NR2D, NR3A und NR3B. NMDA-Rezeptoren haben eine tetramere Struktur.

Der NMDA-Rezeptor weist eine Vielzahl an Bindungsstellen auf, an denen Substanzen den Rezeptor inhibieren, aktivieren oder seine Funktion steigem können. Unter Ruhebedingungen ist der NMDA-Rezeptor geschlossen und durch Magnesium-Ionen blockiert (spannungsabhängiger Magnesiumblock bei negativem Membranpotenzial). Das Ausmaß des Magnesium-Effekts ist jedoch vom Typ des NMDA-Rezeptors abhängig und bei Keratinozyten aufgrund des Subtyps NMDAR2D als gering einzustufen (Fischer et al. Arch Dermatol Res. 2005, im Druck). Erst nach der Stimulation durch L-Glutamat oder einen Agonisten, wie N-Methyl-D-Aspartat, kommt es zur Öffnung des Kanalproteins mit nachfolgendem intrazellulärem Calciumeinstrom.

Erfindungsgemäß wird ein NMDA-Rezeptor-Agonist zur Aktivierung des NMDA-Rezeptors eingesetzt. Dies erfolgt durch die Bindung des Wirkstoffs an extrazelluläre Stellen des NMDA-Rezeptors, wodurch der Rezeptor aktiviert wird. Unter der Aktivierung versteht man den Vorgang der Öffnung des transmembranösen Calciumkanals, welcher ein integraler Bestandteil des NMDA-Rezeptors ist. Hierdurch können Ca²⁺-Ionen aus dem extrazellulären Raum in die Zelle eindringen, wodurch eine Erhöhung der intrazellulären Calciumkonzentration bewirkt wird. Die dadurch bewirkte Erhöhung der intrazellulären Calciumkonzentration fördert die Differenzierung der Keratinozyten.

Erfindungsgemäß können grundsätzlich alle Substanzen als NMDA-Rezeptor-Agonisten verwendet werden, die eine ausreichende Selektivität für den Rezeptor aufweisen bei gleichzeitig geringer Exotoxizität bzw. Toxizität bei systemischen Behandlungs-Varianten. Beispiele für NMDA-Rezeptor-Agonisten, die erfindungsgemäß verwendet werden können, sind, neben N-Methyl-D-Aspartat (NMDA) selbst, L-Glutamat, L-Aspartat, ein Chinolat, ein Homocysteat, D-Aspartat, D-Glutamat, trans-1-Aminocyclobutan-1,3-dicarbonsäure, (±)-1-Aminocyclopentan-cis-1,3-dicarbonsäure, 2-Carboxy-3-carboxymethylchinolin, L-Cysteinsulfinsäure, 3-Carboxymethylpyridine-2-carbonsäure, α-Amino-(3-hydroxy-5- isoxazolyl)essigsäure, 4-Methoxy-7-nitroindolinyl-caged-L-glutamat (S)-α-Amino-2,3-dihydro-4-methoxy-7-nitro-δ-oxo-1H-indole-1-pentansäure, Pyridin-2,3-dicarbonsäure, (RS)-(Tetrazol-5-yl)glycin und Derivate davon.

Auf der NR1 Untereinheit des NMDA-Rezeptors befindet sich eine extrazelluläre Glycin-Co-Agonist-Bindungsstelle. Die Bindung eines entsprechenden Co-Agonisten an dieser Stelle verstärkt die Wirkung des NMDA-Rezeptor-Agonisten am gleichnamigen Rezeptor hinsichtlich seiner Aktivierung. Bevorzugt ist daher eine pharmazeutische Zusammensetzung, welche sowohl einen NMDA-Rezeptor-Agonisten als auch einen NMDA-Rezeptor Co-Agonisten enthält, wobei letzterer an die Glycin-Co-Agonist Bindungsstelle bindet. Dadurch wird die Wirkung des Medikaments hinsichtlich der Erhöhung der intrazellulären Calciumkonzentration insgesamt verstärkt.

Erfindungsgemäß kommen als NMDA-Rezeptor Co-Agonist alle Substanzen in Frage, die eine ausreichende Selektivität an dem Rezeptor aufweisen bei gleichzeitig geringer Exotoxizität, bzw. Toxizität bei systemischen Behandlungs-Varianten. Hierzu zählen unter anderem Glycin, D-Serin, L-Alanin, D-Cycloserin, R(+)-3-amino-1-hydroxypyrrolid-2-on, R(+)-cis-β-methyl-3-amino-1-hydroxypyrrolid-2-on, ein Polyamin, wie beispielsweise Spermidin, und Derivate davon.

Auf der NR2 Untereinheit des NMDA-Rezeptors befindet sich eine extrazelluläre Bindungsstelle für Zn²⁺-lonen. Die Bindung von Zink-Ionen an dieser Stelle bewirkt eine Inhibierung der Aktivität des NMDA-Rezeptors, wodurch der Fluß an Calcium-lonen in die Zelle gehemmt wird. Extrazelluläre Zn²⁺-lonen, welche an den NMDA-Rezeptor binden können, mindern daher die Wirkung des erfindungsgemäßen Medikaments. Besonders bevorzugt ist daher die Verwendung eines Medikaments, welches einen NMDA-Rezeptor-Agonisten, einen NMDA-Rezeptor-Co-Agonisten und ein Zn²⁺-chelatisierendes Agens enthält.

Als Zn²⁺-chelatisierendes Agens werden erfindungsgemäß vorzugsweise Dithiotreithol, Glutathion, CaEDTA und Derivate davon verwendet.

Als differenzierungsfördernde Substanzen werden erfindungsgemäß vorzugsweise Retinoide, Vitamin D in all seinen Isoformen, 1,25-Dihydroxycholecalciferol (Calcitriol), 7-Dehydrocholesterin, Zytokine, Wachstumsfaktoren, und Derivate davon verwendet. Unter den Wachstumsfaktoren werden Epidermis Wachstumsfaktor (Epidermal growth factor; EGF) und Transformierender Wachstumsfaktor-α (Transforming growth factor; TGF-α) bevorzugt verwendet.

In einer bevorzugten Ausführungsform wird mindestens ein N-Methyl-D-Aspartat-Rezeptor-Agonist mit mindestens einem N-Methyl-D-Aspartat-Rezeptor-Co-Agonisten kombiniert. Dabei kann jeder der oben aufgeführten N-Methyl-D-Aspartat-Rezeptor-Agonisten mit jedem der oben aufgeführten N-Methyl-D-Aspartat-Rezeptor-Co-Agonisten beliebig kombiniert werden. In einer weiteren bevorzugten Ausführungsform wird mindestens ein N-Methyl-D-Aspartat-Rezeptor-Agonist mit mindestens einem Zn²⁺-chelatisierendes Agens kombiniert. Dabei kann jeder der oben aufgeführten N-Methyl-D-Aspartat-Rezeptor-Agonisten mit jedem der oben aufgeführten Zn²⁺-chelatisierenden Agenzien beliebig kombiniert werden. Es ist weiterhin bevorzugt, mindestens einen N-Methyl-D-Aspartat-Rezeptor-Agonisten mit mindestens einem differenzierungsfördemden Agens zu kombinieren. Auch hierbei kann jeder der oben aufgeführten N-Methyl-D-Aspartat-Rezeptor-Agonisten mit jedem der oben aufgeführten differenzierungsfördemden Agenzien beliebig kombiniert werden.

In einer ganz bevorzugten Ausführungsform wird mindestens ein N-Methyl-D-Aspartat-Rezeptor-Agonist mit mindestens einem N-Methyl-D-Aspartat-Rezeptor-Co-Agonisten und mindestens einem Zn²⁺-chelatisierendes Agens und/oder mindestens einem differenzierungsfördemden Agens kombiniert. Dabei jeder der oben aufgeführten N-Methyl-D-Aspartat-Rezeptor-Agonisten mit jedem der oben aufgeführten N-Methyl-D-Aspartat-Rezeptor-Co-Agonisten und jedem der oben aufgeführten Zn²⁺-chelatisierenden oder differenzierungsfördernden Agenzien beliebig kombiniert werden.

Für die Applikation kommen alle pharmazeutische Darreichungsformen in Betracht, welche die gewünschte Wirkung herbeiführen, vorausgesetzt die sonstigen Bestandteile der Zusammensetzung gehen keine unerwünschten Wechselwirkungen oder Reaktionen mit der Haut oder den Wirkstoffen selbst ein. Bevorzugt ist die topische Anwendung, wobei das Medikament beispielsweise in Form einer halbfesten Zusammensetzung, wie einer Salbe, einer Creme oder eines Gels, oder in Form einer Lösung verabreicht werden kann. Die genaue Applikationsform richtet sich nach der Art und dem Schweregrad der Erkrankung sowie nach den in der Zusammensetzung enthaltenen Wirkstoffen.

Bei den halbfesten Zubereitungen kann es sich beispielsweise um hydrophobe, wasseraufnehmende oder hydrophile Salben handeln. Cremes umfassen mehrphasige Zubereitungen und können hydrophob, amphiphil oder hydrophil sein. Dagegen sind Gele durch Quellmittel gelierte Flüssigkeiten, die nach Auftragung durch das Verdunsten des Wassers eine kühlende und lindernde Wirkung aufweisen. Dabei bilden sie ein Gerüst, in dem die Flüssigkeit samt dem darin gelösten Wirkstoff an der Oberfläche immobilisiert wird. Gele können dabei sowohl hydrophob (Oleogele) als auch hydrophil (Hydrogele) wirken. In bestimmten Fällen ist die Verwendung einer Paste angezeigt, die zwar eine ähnliche Grundlage wie die Salben besitzt, jedoch zusätzlich einen großen Anteil an fein dispergiertem Pulver enthält.

Die oben aufgeführten Beispiele für halbfeste Zubereitungen können eine Reihe an pharmazeutisch akzeptablen Hilfsmitteln enthalten. In Frage kommen hierfür Feuchthaltemittel, wie Sorbitol, Glycerol oder Propylenglykol; Penetrationsverbesserer, wie Dimethylsulfoxid oder Ölsäureoleylester; Konservierungsmittel, wie p-HydroxyBenzoesäureester, Sorbinsäure, Benzylalkohol oder Ascorbinsäureester; und Antioxidantien, wie Butylhydroxyanisol, Vitamin E oder Vitamin C.

Die erfindungsgemäße Zusammensetzung kann zur Behandlung aller Dermatosen verwendet werden, die mit einem gestörten Verhomungsmuster der Haut zusammenhängen. Allgemein zählen hierzu entzündliche Erkrankungen der Haut sowie alle Präkanzerosen, Kanzerosen und benignen Erkrankungen. Insbesondere können verschiedene Ekzemkrankheiten (Dermatitis), wie atopisches Ekzem (Neurodermitis), allergisches Kontaktekzem, toxisch-irritatives Ekzem, Seborrhoisches Ekzem und Nummuläres Ekzem; entzündliche Erkrankungen der Haut, wie Schuppenflechte (Psoriasis), Schuppenröschen (Pityriasis rosea), Pityriasis rubra pilaris, Lichen ruber, Pytiriasis lichenoides; vererbliche Hautkrankheiten, wie Ichthyosen und hereditäre Palmoplantarkeratosen; Präkanzerosen, wie aktinische Keratosen; gut- und bösartige Hauttumore, wie Porokeratosen, spinozelluläres Karzinom, Basaliom, Fibrom oder seborrhoische Keratose behandelt werden.

Die erfindungsgemäße Zusammensetzung enthält den NMDA-Rezeptor-Agonisten üblicherweise in einer Konzentration von 1 µM - 100 mM in den flüssigen Zubereitungen bzw. in einer Konzentration von 0,01 % - 10 % in den halbfesten Zubereitungsformen. Als systemisches Medikament beträgt die Einzeldosis geeigneterweise 1 µg bis 100 mg.

Bevorzugt enthält das Medikament zusätzlich einen NMDA-Rezeptor-Co-Agonisten in einer Konzentration von 10 nM - 100 mM in den flüssigen Zubereitungen bzw. in einer Konzentration von 0,01 % - 10 % in den halbfesten Zubereitungsformen. Als systemisches Medikament beträgt die Einzeldosis 1 µg/kg bis 100 mg/kg Körpergewicht eines NMDA-Rezeptor Co-Agonisten.

Noch bevorzugter sind Ausführungen, welche zusätzlich ein Zn²⁺-chelatisierendes Agens in einer Konzentration von 10 nM - 100 mM in den flüssigen Zubereitungen bzw. in einer Konzentration von 0,01 % - 10 % in den halbfesten Zubereitungsformen enthalten. Als systemisches Medikament beträgt die Einzeldosis 1 µg/kg bis 100 mg/kg eines Zn²⁺-chelatisierendes Agens.

Besonders bevorzugt sind Ausführungen, welche zusätzlich ein Ca²⁺-Ionen spendendes Agens in einer auf das Calcium bezogenen Konzentration von 10 nM - 100 mM in den flüssigen Zubereitungen bzw. in einer Konzentration von 0,01 % - 10 % in den halbfesten Zubereitungsformen enthalten. Als systemisches Medikament beträgt die Einzeldosis 1 µg/kg bis 100 mg/kg eines Ca²⁺-Ionen spendenden Agens.

Ganz besonders bevorzugt sind Ausführungen, welche zusätzlich eine differenzierungsfördernde Substanz in einer Konzentration von 10 nM - 100 mM in den flüssigen Zubereitungen bzw. in einer Konzentration von 0,01 % - 10 % in den halbfesten Zubereitungsformen enthalten. Als systemisches Medikament beträgt die Einzeldosis einer differenzierungsfördernden Substanz 1 µg/kg bis 100 mg/kg.

Das folgende Beispiel erläutert die Erfindung.

### Beispiel

Die Wirkung des NMDA-Rezeptor-Agonisten N-Methyl-D-Aspartat auf die intrazelluläre Calciumkonzentration von Keratinozyten wurde bei menschlichen Hautzellen (HaCaT-Zellen und NHEK: normal human epidermal keratinocyte) (Fischer et al. Experimental Dermatology 2004, 13, 512-519). In weiterführenden Experimenten wurde in gleicher Weise die Wirkung des physiologischen Agonisten am NMDA-Rezeptor, L-Glutamat, auf die intrazelluläre Calciumkonzentration von Keratinozyten untersucht. Dabei wurden L-Glutamat Lösungen mit Konzentrationen zwischen 1µM und 1000µM auf NHEK Zellkulturen appliziert.

Die Messung der intrazellulären Calciumkonzentrationen von NHEK erfolgte als Fluoreszenzmessung mittels Laser Scanning Mikroskopie. Der Untersuchungszeitraum erstreckte sich über insgesamt 10 Minuten, wobei die Effektoren über den gesamten Untersuchungszeitraum auf den Zellen belassen wurden.

Für die Fluoreszenzmessungen wurden die NHEK auf 40mm Deckgläschen (Helmut Saur Laborbedarf, Reutlingen, Deutschland) in 60-mm-Kulturschalen gezogen. Die Beladung der Zellen mit fluoreszenzmarkierten Farbstoffen und die Messungen erfolgten nach Lipp und Niggli (Lipp und Niggli. Cell Calcium 1993, 14, 359-372) in Hepes gepufferte Salzlösung (HBS). HBS, HEPES-gepufferte Glucose-Elektrolytlösung wurde in dreifach destilliertem Wasser hergestellt und enthielt (in mM): Glucose 5, NaCl 140, KCI 5, EDTA 0,2, freies Ca²⁺ 1, freies Mg²⁺ 1 und HEPES 5. Der pH lag bei 7,4. Als Fluoreszenzfarbstoffe werden Fura Red (2µmol/l), Fluo-3 (1µmol/l) und 3µl/ml Pluronic (20 % in DMSO) in HBS verwendet.

Die zu untersuchenden Zellen wurden für 30 Minuten in den genannten Fluoreszenzfarbstoffen inkubiert. Anschließend erfolgte eine Inkubation in farbstofffreiem HBS für weitere 30 Minuten. Die so vorbereiteten Zellen wurden in eine POC-Kammer überführt, mit 1ml HBS überschichtet und nach Erreichen einer stabilen Grundlinie unter dem Mikroskop mit den verschiedenen Effektoren versehen. Die Fluoreszenzmessungen wurden mit einem konfokalen Laserscanning-Mikroskop (Leica DM IRE2, Leica, Bensheim, Deutschland) mit einem HCX PL APO CS 40x 1.25 Oil-Objektiv (Leica) bei Raumtemperatur durchgeführt. Die Anregung erfolgte mit einem Argonlaser (488 nm). Die Emissionen von 510 - 540 nm und von 640 - 670 nm wurden erfasst, die Messergebnisse als zwei monochrome mikroskopische Bilder gespeichert und einer rechnerischen Verarbeitung zugeführt. Die intrazellulären Calciumkonzentrationen wurden nicht direkt gemessen, sondern errechneten sich aus der relativen Veränderung der Fluoreszenzintensitäten. Dabei führte ein Anstieg der intrazellulären Calciumkonzentration zu einer Zunahme der grünen Fluoreszenz (525 nm) und einer gleichzeitigen Abnahme der roten Fluoreszenz (660 nm). Die Berechnung der intrazellulären Calciumkonzentrationen erfolgte dann über das Verhältnis der Fluoreszenzintensitäten.

Ein Anstieg der intrazellulären Calciumkonzentration um mindestens 18% des Ausgangswertes wurde als positive Reaktion definiert (3SD der spontanen intrazellulären Ca-Schwankung). Zur Erfassung des Einflusses der eingesetzten Effektoren auf die Calciumkonzentration des Keratinozyten wurde zunächst für jede Zelle separat ein Ausgangswert bestimmt, der sich aus dem Mittelwert von drei Messungen vor der Zugabe der genannten Effektoren errechnete. Dieser Mittelwert wurde gleich 100% gesetzt. Alle weiteren Messwerte wurden hierzu in Relation gesetzt. Die absolute Zahl der reaktiven Zellen (Anstieg der intrazellulären Calciumkonzentration >18%) während des gewählten Beobachtungszeitraums von zehn Minuten wurde mit der absoluten Zahl der nichtreaktiven Zellen verglichen. Die statistische Auswertung erfolgte mit Fisher's exaktem Test.

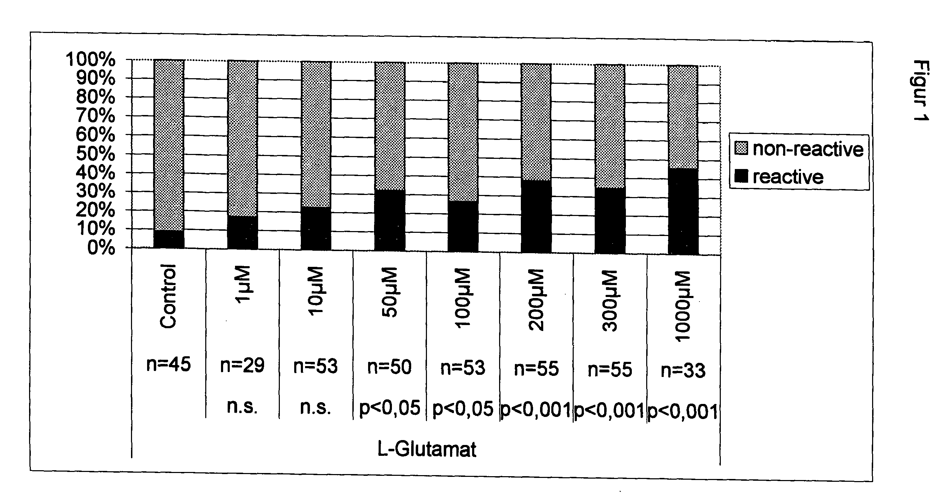

Die Ergebnisse der Versuchsreihe sind in Figur 1 zusammengefasst. Die variable "*n*" steht für die Zahl der untersuchten Zellen. n.s. = nicht signifikant; p= Irrtumswahrscheinlichkeit.

In der Zellkultur steigt bei NHEK ab einer L-Glutamat-Konzentration von 50µM die Anzahl reaktiver Zellen signifikant an. Dieser Effekt ist durch eine weitere Dosissteigerung nicht signifikant zu verbessern. Die prozentual meisten reaktiven Zellen finden sich aber bei 1000µM L-Glutamat.

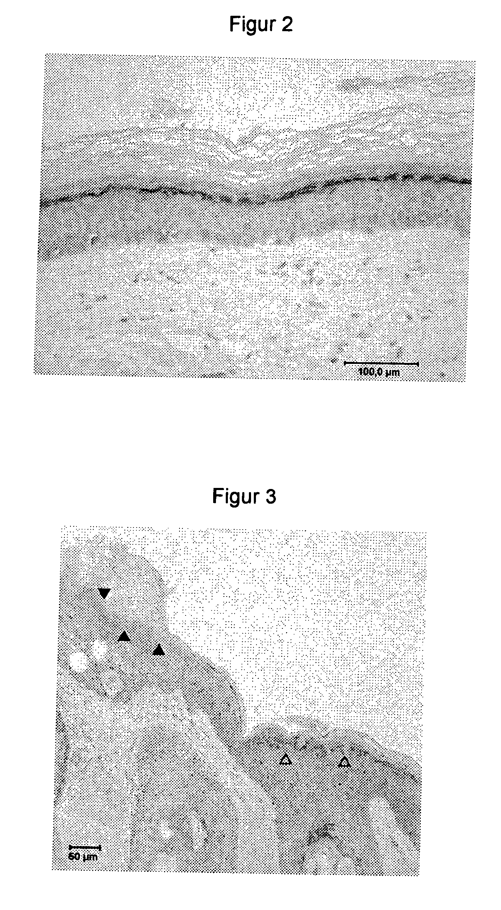

Zur Vorbereitung des Einsatzes von NMDA-Rezeptor-Agonisten bei verschiedenen Dermatosen ist eine immunhistochemische Nachweismethode etabliert, die die Expression der konstanten Rezeptorkomponente NMDAR1 in der Haut nachweist. Hierfür wird ein monoknonaler IgG2a-Anti-NR1 Mausantikörper (Clone 54.1, BD PharMingen, Heidelberg, Deutschland) verwandt. Dieser Antikörper ist gegen ein Fusionsprotein (aa 660-811 der NR1 Domäne) gerichtet, welches den intrazellulären Loop der transmembranösen Regionen III und IV von NMDAR1 darstellt (Siegel et al. Proc Natl Acad Sci USA 1994, 91: 564-568). Figur 2 (Immunhistochemische Darstellung von NMDA-Rezeptoren (NMDAR1) in gesunder menschlicher Haut) zeigt die Expression von NMDAR1 in gesunder Epidermis mit besonderer Betonung des Stratum granulosum. Figur 3 (Aktinische Keratose - immunhistochemische Darstellung von NMDAR1. Reduktion von NMDAR1 beim Auftreten einer Parakeratose (▲). (△= normale Verteilung von NMDAR1)) stellt eine aktinische Keratose dar, bei der die Expression beim Auftreten einer (pathologischen) parakeratotischen Verhornung vermindert ist.

## Patentansprüche

1. Verwendung mindestens eines N-Methyl-D-Aspartat-Rezeptor-Agonisten zur Herstellung eines Medikaments zur Behandlung von Dermatosen, welche durch ein gestörtes Verhomungsmuster charakterisiert sind.

2. Verwendung nach Anspruch 1, wobei der N-Methyl-D-Aspartat-Rezeptor-Agonist aus der Gruppe, bestehend aus L-Glutamat, L-Aspartat, N-Methyl-D-Aspartat, einem Chinolat, einem Homocysteat, D-Aspartat, D-Glutamat, trans-1-Aminocyclobutan-1,3-dicarbonsäure, (±)-1-Aminocyclopentan-cis-1,3-dicarbonsäure, 2-Carboxy-3-carboxymethylchinolin, L-Cysteinsulfinsäure, 3-Carboxymethylpyridine-2-carbonsäure, α-Amino-(3-hydroxy-5- isoxazolyl)essigsäure, 4-Methoxy-7-nitroindolinyl-caged-L-glutamat (S)-α-Amino-2,3-dihydro-4-methoxy-7-nitro-δ-oxo-1H-indole-1-pentansäure, Pyridin-2,3-dicarbonsäure, (RS)-(Tetrazol-5-yl)glycin und Derivaten davon, gewählt wird.

3. Verwendung nach Anspruch 1 oder 2, wobei das Medikament zusätzlich mindestens einen N-Methyl-D-Aspartat-Rezeptor-Co-Agonisten enthält.

4. Verwendung nach Anspruch 3, wobei der N-Methyl-D-Aspartat-Rezeptor-Co-Agonist aus der Gruppe, bestehend aus Glycin, D-Serin, L-Alanin, D-Cycloserin, R(+)-3-amino-1-hydroxypyrrolid-2-on, R(+)-cis-β-methyl-3-amino-1-hydroxypyrrolid-2-on, einem Polyamin, und Derivaten davon, gewählt wird.

5. Verwendung nach einem der Ansprüche 1 bis 4, wobei das Medikament zusätzlich mindestens ein Zn²⁺-chelatisierendes Agens enthält.

6. Verwendung nach Anspruch 5, wobei das Zn²⁺-chelatisierende Agens aus der Gruppe, bestehend aus Dithiotreithol, Glutathion, CaEDTA, und Derivaten davon, gewählt wird.

7. Verwendung nach einem der Ansprüche 1 bis 6, wobei das Medikament zusätzlich mindestens ein Ca²⁺-lonen spendendes Agens enthält.

8. Verwendung nach einem der Ansprüche 1 bis 7, wobei das Medikament zusätzlich mindestens ein differenzierungsfördemdes Agens enthält.

9. Verwendung nach Anspruch 8, wobei das differenzierungsfördernde Agens aus der Gruppe, bestehend aus Retinoiden, Vitamin D, 1,25-Dihydroxycholecalciferol (Calcitriol), 7-Dehydrocholesterin, Zytokine, Wachstumsfaktoren und Derivaten davon, gewählt wird.

10. Verwendung nach einem der Ansprüche 1 bis 9, wobei das Medikament topisch oder subkutan appliziert wird.

11. Pharmazeutische Zusammensetzung, welche mindestens einen N-Methyl-D-Aspartat-Rezeptor-Agonisten und mindestens einen Bestandteil, gewählt aus einem N-Methyl-D-Aspartat-Rezeptor Co-Agonisten, einem Zn²⁺-chelatisierendes Agens und einem differenzierungsfördernden Agens, enthält.

12. Pharmazeutische Zusammensetzung nach Anspruch 11, wobei der N-Methyl-D-Aspartat-Rezeptor-Agonist aus der Gruppe, bestehend aus L-Glutamat, L-Aspartat, N-Methyl-D-Aspartat, einem Chinolat, einem Homocysteat, D-Aspartat, D-Glutamat, trans-1-Aminocyclobutan-1,3-dicarbonsäure, (±)-1-Aminocyclopentan-cis-1,3-dicarbonsäure, 2-Carboxy-3-carboxymethylchinolin, L-Cysteinsulfinsäure, 3-Carboxymethylpyridine-2-carbonsäure, α-Amino-(3-hydroxy-5- isoxazolyl)essigsäure, 4-Methoxy-7-nitroindolinyl-caged-L-glutamat (S)-α-Amino-2,3-dihydro-4-methoxy-7-nitro-δ-oxo-1H-indole-1-pentansäure, Pyridin-2,3-dicarbonsäure, (RS)-(Tetrazol-5-yl)glycin und Derivaten davon, gewählt wird.

13. Pharmazeutische Zusammensetzung nach Anspruch 11 oder 12, wobei der N-Methyl-D-Aspartat-Rezeptor-Co-Agonist aus der Gruppe, bestehend aus Glycin, D-Serin, L-Alanin, D-Cycloserin, R(+)-3-amino-1-hydroxypyrrolid-2-on, R(+)-cis-β-methyl-3-amino-1-hydroxypyrrolid-2-on, einem Polyamin, und Derivaten davon, gewählt wird.

14. Pharmazeutische Zusammensetzung nach einem der Ansprüche 11 bis 13, wobei das Zn²⁺-chelatisierende Agens aus der Gruppe, bestehend aus Dithiotreithol, Glutathion, CaEDTA, und Derivaten davon, gewählt werden.

15. Pharmazeutische Zusammensetzung nach einem der Ansprüche 11 bis 14, welche zusätzlich mindestens ein Ca²⁺-lonen spendendes Agens enthält.

16. Pharmazeutische Zusammensetzung nach einem der Ansprüche 11 bis 15, in welcher das differenzierungsfördernde Agens aus der Gruppe, bestehend aus Retinoiden, Vitamin D, 1,25-Dihydroxycholecalciferol (Calcitriol), 7-Dehydrocholesterin, Zytokine, Wachstumsfaktoren und Derivaten davon, gewählt wird.
